# DEMANDE DE BREVET EUROPEEN

(11) **EP 4 763 053 A1**
(43) Date de publication de la demande: **24.06.2026**
(21) Numéro de dépôt: 24307225.3
(22) Date de dépôt: 20.12.2024
(51) Int. Cl.: A61B 5/00, A61B 5/01, G01K 13/20

(54) **DISPOSITIF D'ANALYSE PAR THERMOGRAPHIE POUR LA DÉTECTION D'ANOMALIES DANS LES TISSUS MAMMAIRES**

(71) Demandeur: École Nationale Supérieure de Mécanique et des Microtechniques, 25000 Besançon (FR); Centre National de la Recherche Scientifique, 75016 Paris (FR); Hopital Nord Franche-Comté, 90400 Trévenans (FR)
(72) Inventeur: AL MASRY, Zeina, 25000 Besançon (FR); ZERHOUNI, Noureddine, 25000 Besançon (FR); MARIONNET, François, 25000 Besançon (FR); DEVALLAND, Christine, 90000 Belfort (FR); ROUGEOT, Patrick, 25000 Besançon (FR)
(74) Mandataire: IPAZ

(57) **Abrégé**

L'invention concerne un Dispositif d'analyse par thermographie par contact cutané (1) pour la détection d'anomalies dans les tissus mammaires, dit dispositif (1). Le dispositif (1) comprend : deux réseaux (2) de capteurs de température (3) étant destinés à être en contact avec la peau d'un sein respectif et étant agencés pour paver de manière homogène chacun des seins, et une unité d'acquisition (4) de données de température au cours du temps, provenant des capteurs de température, agencée pour communiquer avec une unité de traitement (5). L'unité de traitement (5) est agencée pour calculer, à partir des données de température, un écart de température entre les deux seins en fonction du temps, sélectionner, à partir des données de température en fonction du temps, un modèle de traitement de données approprié, et déterminer une information représentative de la présence ou non d'une anomalie par traitement des données de température avec le modèle de traitement sélectionné.

## Description

### Domaine technique

La présente invention appartient au domaine de la détection d'anomalies dans les tissus mammaires.

L'invention porte, en particulier, sur la détection d'anomalies dans les tissus mammaires par thermographie par contact cutané.

L'invention vise notamment à la détection de tissus tumoraux au sein de tissus mammaires.

L'invention se rapporte, en outre, à un soutien pour la détection d'anomalies mammaires par thermographie par contact cutané.

### État de la technique

Les tissus tumoraux se distinguent des tissus sains environnants par leur température du fait de l'inflammation causée et de l'angiogenèse.

On connait dans l'état de la technique des soutiens-gorge commerciaux pour la détection de tissus cancéreux par thermographie.

A titre d'exemple, le soutien-gorge « iTBra » propose une détection de tissus tumoraux basée sur la mesure de température au cours d'un cycle circadien. Ce modèle nécessite la mesure de données de température pendant au moins un cycle circadien (au moins 24 heures). La température du tissu mammaire sain évolue selon les rythmes circadiens alors que la température de tissu cancéreux n'y est pas dépendante.

On connait également le soutien-gorge « evabra » propose une détection de tissus tumoraux basée sur la mesure de température en continu. La mesure de température doit être ininterrompue. La mesure de température doit être mise en oeuvre pendant une durée minimale de 60 à 90 minutes.

Un but de la présente invention est de remédier à au moins un des inconvénients de l'état de la technique.

Un autre but de l'invention est de proposer un soutien-gorge pour et un procédé de détection d'anomalie dans des tissus mammaires :
- par mesure en contact cutané, et/ou
- par thermographie, et/ou
- de détecter la présence d'anomalies malignes et/ou d'anomalie bénignes, et/ou
- de détecter des anomalies, en particulier la présence de tissus tumoraux, à un stade précoce, et/ou
- de discriminer la présence d'une anomalie maligne d'une anomalie bénigne, et/ou
- permettant une réponse fiable, c'est-à-dire avec peu de faux positif, même pour de faibles variation de températures,
- nécessitant un temps d'acquisition des données de température réduit et/ou inférieur aux procédés et soutiens-gorge de l'état de la technique, et/ou
- nécessitant un temps de traitement des données de température réduit.

### Exposé de l'invention

À cet effet, l'invention concerne un dispositif d'analyse par thermographie pour la détection d'anomalies dans les tissus mammaires, dit dispositif. Le dispositif comprend :
- deux réseaux de capteurs de température étant destinés à être en contact avec la peau d'un sein respectif et étant agencés, disposés ou répartis pour paver de manière homogène chacun des seins,
- une unité d'acquisition de données de température au cours du temps, provenant des capteurs de température, agencée pour communiquer avec une unité de traitement.

L'unité de traitement est agencée pour :
- calculer, à partir des données de température, un écart de température entre les deux seins en fonction du temps,
- sélectionner, de préférence au moyen de l'unité de traitement, à partir des données température, ou partir des données de température et de données phénotypiques, un profil de température correspondant, approprié, représentatif et/ou associé aux données de température, et/ou
- sélectionner, à partir de l'écart de température en fonction du temps calculé et/ou du profil de température sélectionné, un modèle de traitement de données approprié, adapté ou représentatif,
- déterminer une information représentative de la présence ou non d'une anomalie par traitement des données de température avec le modèle de traitement sélectionné.

De préférence, l'unité de traitement et/ou, respectivement, l'unité d'acquisition est agencée et/configurée pour communiquer et/ou être connectée, en liaison filaire ou sans fil, à l'unité d'acquisition et/ou, respectivement, à l'unité de traitement.

L'unité de traitement peut être une unité distante. De préférence l'unité de traitement et/ou l'unité d'acquisition est agencée et/ou configurée pour communiquer avec l'unité d'acquisition.

L'unité de traitement et/ou l'unité d'acquisition peuvent comprendre un émetteur/récepteur. L'unité de traitement et/ou l'unité d'acquisition peuvent comprendre un émetteur/récepteur agencé et/ou configuré pour communiquer et/ou transmettre :
- les données de température provenant des capteurs, et/ou
- les données traitées par l'unité de traitement, c'est-à-dire l'information représentative de la présence ou non d'une anomalie.

De préférence, l'unité de traitement est agencée, en outre, pour, à partir des données de température et/ou à partir de l'écart de température calculé, déterminer une signature statistique de l'écart de température entre les deux seins.

De préférence, l'unité de traitement est agencée, en outre, pour sélectionner, à partir de la signature statistique de l'écart de température déterminée et du profil de température sélectionné, le modèle de traitement de données approprié.

De préférence, chaque réseau de capteurs de température comprend un support flexible d'un seul tenant sur lequel sont disposés les capteurs de température.

De préférence, le support flexible comprend un ensemble de languettes ou segments distinct(e)s de géométrie actinomorphe.

De préférence, le support flexible comprend une portion centrale destinée à être positionnée sur la papille mammaire d'un sein.

De préférence, les languettes s'étendent radialement depuis une portion centrale du support. De préférence, les languettes sont agencées pour et/ou destinées à s'étendre radialement depuis la papille mammaire vers la périphérie du sein.

De préférence, chacune des languettes est séparée des deux languettes adjacentes par deux fentes situées de part et d'autre de chaque languette.

De préférence, la portion centrale est agencées pour et/ou destinées à s'étendre principalement radialement depuis la papille mammaire vers la périphérie du sein

De préférence, un ou chaque réseau de capteurs comprend des capteurs de température agencés pour et/ou destinés à être positionnés sur le sein, la papille mammaire et/ou aux abords du sein.

De préférence, au moins une partie, de préférence seulement une partie, de préférence encore tous les capteurs à l'exception des agencés pour et/ou destinés à être positionnés sur la papille mammaire et/ou aux abords du sein, des capteurs de température sont répartis en losange sur le support flexible.

De préférence, chacun des quatre sommets d'un losange correspond à un capteur de température.

De préférence, pour chaque losange considéré, trois capteurs de température sont disposés sur une languette donnée et un capteur de température est disposé sur une languette adjacente à la languette donnée.

De préférence, pour chaque capteur de chaque languette, trois capteurs de température sont disposés sur une languette donnée et un capteur de température est disposé sur une languette adjacente à la languette donnée.

Selon l'invention, il est proposé également un soutien-gorge pour la détection d'anomalies dans les tissus mammaires par thermographie comprenant le dispositif selon l'invention.

Le soutien-gorge selon l'invention comprend, en outre, deux bonnets.

Chaque réseau de capteurs de température destiné à être en contact avec la peau d'un sein respectif et à paver de manière homogène un des seins est disposé à l'intérieur d'un des bonnets respectifs.

Chacun des bonnets est agencé pour isoler thermiquement les capteurs de température de l'environnement extérieur.

Alternativement, le dispositif d'analyse peut être décrit comme formant ou étant agencé pour former le soutien-gorge l'invention.

Selon l'invention, il est proposé également un procédé de détection d'anomalies dans les tissus mammaires par thermographie, dit procédé.

Le procédé comprend les étapes consistant à :
- calculer, au moyen de l'unité de traitement, un écart de température entre les deux seins en fonction du temps à partir de données de température de chacun des seins,
- sélectionner, au moyen de l'unité de traitement, à partir des données température, ou partir des données de température et de données phénotypiques, un profil de température sélectionné, et/ou
- sélectionner, au moyen de l'unité de traitement, à partir de l'écart de température en fonction du temps calculé et du profil de température sélectionné, un modèle de traitement de données de température approprié,
- déterminer une information représentative de la présence ou non d'une anomalie par traitement, au moyen de l'unité de traitement, des données de température avec le modèle de traitement sélectionné.

Le modèle de traitement de données, dit modèle, peut être choisi ou sélectionné à partir ou parmi un ensemble de modèles dans une base de données.

De préférence, le profil de température est sélectionné ou choisi à partir d'un ensemble de profils de température, ou à partir d'un ensemble de profils de température et d'un ensemble de profils phénotypiques, dans une base de données.

De préférence, chaque profil de température est fonction :
- de données de données de température d'un ou des seins, et/ou
- de données phénotypiques.

De préférence, le procédé comprend une étape d'acquisition, au moyen d'une unité d'acquisition, des données de température.

De préférence, les données de température sont mesurées au moyen des deux réseaux de capteurs de température.

Selon l'invention, le procédé peut ne pas comprendre étape d'acquisition. Le procédé peut être mis en oeuvre à partir de données de température de chacun des seins fournies, transmises ou reçues (par exemple par un élément externe ou distant) ou stockées (par exemple dans une base de données), ou encore à partir de données simulées (pouvant être des données stockées ou transmises).

L'étape d'acquisition peut être mise en oeuvre indépendamment des autres étapes du procédé.

De préférence, le procédé comprend, en outre, une étape de détermination, à partir des données de température et/ou à partir de l'écart de température calculé, d'une signature statistique de l'écart de température entre les deux seins.

De préférence, la sélection du modèle traitement de données de température est mise en oeuvre à partir de la signature statistique de l'écart de température déterminée et du profil de température sélectionné.

De préférence, l'étape de sélection du modèle de traitement de données est mise en oeuvre, entre autres, à partir d'une variation de l'écart de température entre les deux seins dans le temps et du profil de température sélectionné.

De préférence, la signature statistique et/ou l'écart de température correspond ou est représentative d'une variation de l'écart de température entre les deux seins dans le temps ou au cours du temps ou en fonction du temps.

De préférence, les données de température forment un ensemble de couple de températures correspondant à la température de chacun des seins à un instant t ou à la température moyenne de chacun des seins sur un intervalle de temps donné.

De préférence, les données de température forment ou comprennent une matrice de données pouvant être fonction du temps et d'une position associée ou relative (de préférence sur le sein et/ou à proximité du sein) à chaque donnée de température individuelle. De préférence, les positions de chaque donnée de température correspondent à la position de chaque capteur de température disposés pour être en contact sur une portion ou un point différent de chaque sein, de la papille mammaire et/ou des abords du sein. De préférence, les données de température forment ou comprennent une matrice distincte pour chacun des deux seins.

De préférence, l'écart de température et/ou la signature statistique est déterminée par comparaison des données de température d'un des seins, choisies comme données de température de référence, avec les données de température de l'autre sein.

De préférence, les données de température de chacun des seins comprennent ou sont dissociées en n ensembles de données de température correspondant chacun à une zone distincte du sein, dit quadrant, où n est un entier.

De préférence, le calcul de l'écart de température et/ou la sélection du modèle de traitement de données et/ou la détermination de la signature statistique est mis en oeuvre à partir ou en fonction ou sur la base de l'ensemble des données de température provenant des n quadrants.

De préférence, le procédé comprend une étape consistant à déterminer, à partir de la signature statistique ou de l'écart de température et du profil de température sélectionné, un ou des indicateurs représentatif(s) d'une tendance de l'écart de température entre les deux seins.

De préférence, l'étape de sélection du modèle de traitement de données de température est mise en oeuvre en à partir du ou des indicateurs déterminés.

De préférence, le traitement des données de température est mis en oeuvre par un modèle d'apprentissage statistique ou profond.

De préférence, le procédé comprend une étape d'apprentissage du modèle sélectionné à partir d'une base de données d'apprentissage. La base de données d'apprentissage comprend :
- des données d'entrée consistant en un ensemble de données de température, chaque ensemble comprenant des données de température provenant de chacun des deux seins d'un même sujet,
- des données de sorties consistant en l'information représentative de la présence ou non d'une anomalie associée à chaque ensemble de données de température.

De préférence, l'étape d'apprentissage est mise en oeuvre, indépendamment, pour chaque modèle de traitement à partir des données d'apprentissage correspondant à l'écart de température et/ou la signature statistique associée et au profil de température sélectionné.

Selon l'invention, il est également proposé un dispositif de traitement de données comprenant des moyens agencés et/ou programmés et/ou configurés pour mettre en oeuvre le procédé selon l'invention.

Selon l'invention, il est également proposé un programme d'ordinateur comprenant des instructions qui, lorsque le programme est exécuté par un ordinateur, conduisent celui-ci à mettre en oeuvre le procédé selon l'invention.

Selon l'invention, il est également proposé un support lisible par ordinateur comprenant des instructions qui, lorsqu'elles sont exécutées par un ordinateur, conduisent celui-ci à mettre en oeuvre le procédé selon l'invention.

L'invention, en particulier le procédé selon l'invention, notamment l'information représentative de la présence ou non d'une anomalie fournie par le procédé selon l'invention, ne se rapporte pas ni ne vise ni ne permet ni ne fournit un diagnostic thérapeutique.

L'information, représentative de la présence ou non d'une anomalie, fournie par le procédé selon l'invention constitue uniquement une information ou un indicateur ne constituant pas et n'ayant pas valeur de diagnostic thérapeutique.

De préférence, le dispositif d'analyse par thermographie pour la détection d'anomalies dans les tissus mammaires et/ou de préférence le soutien-gorge pour la détection d'anomalies dans les tissus mammaires par thermographie selon l'invention convient, de préférence encore est particulièrement adaptée, de manière davantage préférée est conçue et de manière particulièrement avantageuse est spécialement conçue, pour mettre en oeuvre le procédé de détection d'anomalies dans les tissus mammaires par thermographie selon l'invention. Aussi toute caractéristique du dispositif d'analyse par thermographie pour la détection d'anomalies dans les tissus mammaires et/ou du soutien-gorge pour la détection d'anomalies dans les tissus mammaires par thermographie selon l'invention est directement transposable au procédé de détermination de détection d'anomalies dans les tissus mammaires par thermographie et inversement.

### Brève description des FIGURES

L'invention sera mieux comprise à la lecture de la description qui va suivre, donnée uniquement à titre d'exemple non limitatif et faite en se référant aux dessins annexés sur lesquels :
- la FIGURE 1 est une représentation schématique d'un mode de réalisation du dispositif d'analyse par thermographie pour la détection d'anomalies dans les tissus mammaires selon l'invention,
- la FIGURE 2 est une représentation schématique, identique à la FIGURE 1, du dispositif d'analyse par thermographie pour la détection d'anomalies dans les tissus mammaires selon l'invention,
- la FIGURE 3 est une photographie, en vue de face, d'un soutien-gorge comprenant le dispositif d'analyse par thermographie selon l'invention,
- la FIGURE 4 est un graphique illustrant une signature statistique particulière de l'écart de température entre les deux seins.

Il est bien entendu que les modes de réalisation qui seront décrits dans la suite ne sont nullement limitatifs. On pourra notamment imaginer des variantes de l'invention ne comprenant qu'une sélection de caractéristiques décrites par la suite isolées des autres caractéristiques décrites, si cette sélection de caractéristiques est suffisante pour conférer un avantage technique ou pour différencier l'invention par rapport à l'état de la technique antérieure. Cette sélection comprend au moins une caractéristique de préférence fonctionnelle sans détails structurels, ou avec seulement une partie des détails structurels si c'est cette partie qui est uniquement suffisante pour conférer un avantage technique ou pour différencier l'invention par rapport à l'état de la technique antérieure.

En particulier toutes les variantes et tous les modes de réalisation décrits sont combinables entre eux si rien ne s'oppose à cette combinaison sur le plan technique.

Sur les figures et dans la suite de la description, les éléments communs à plusieurs figures conservent la même référence.

### Description détaillée des FIGURES

Selon le mode réalisation non limitatif, il est décrit un exemple de mise en oeuvre du procédé de détection d'anomalies dans les tissus mammaires par thermographie, dit procédé, selon l'invention. Le procédé comprend l'étape consistant à calculer, au moyen d'une unité de traitement 5, un écart de température entre les deux seins à partir de données de température de chacun des seins. L'écart de température entre les deux seins peut, par exemple, être réalisée par comparaison des données de température de chacun des seins.

Le procédé comprend, en outre, l'étape consistant à sélectionner, au moyen de l'unité de traitement 5, à partir des données température, ou partir des données de température et de données phénotypiques, un profil de température correspondant.

Le procédé comprend, en outre, l'étape consistant à sélectionner, au moyen de l'unité de traitement 5, à partir de l'écart de température et du profil de température sélectionné, un modèle de traitement de données de température approprié.

Enfin, le procédé comprend l'étape consistant à déterminer une information représentative de la présence ou non d'une anomalie par traitement, au moyen de l'unité de traitement 5, des données de température avec le modèle de traitement sélectionné.

Selon un premier aspect non limitatif de l'invention, le profil de température est fonction des données de températures. Aussi, le profil de température est sélectionné, à partir des données de températures mesurées, dans une base de données comprenant un ensemble de profils de température.

Selon un second aspect non limitatif de l'invention, le profil de température est fonction des données de températures mesurées et de données phénotypiques. Aussi, le profil de température est sélectionné, à partir des données de températures mesurées et de données phénotypiques, dans une base de données comprenant un ensemble de profils de température.

La sélection du profil de température peut être réalisée par toute méthode, par exemple statistique, connue de l'homme du métier. A titre d'exemple non limitatif, le profil de température peut être sélectionné par analyse de la variance entre les profils de température de la base de données et :
- les données de températures mesurées, ou
- les données de températures mesurées et de données phénotypiques.

Les données phénotypiques sont celles d'un utilisateur du dispositif d'analyse selon l'invention. Les données phénotypiques peuvent comprendre ou se rapporter : à des données morphologiques et/ou à des données subjectives, par exemple liées aux habitudes, comportements, perceptions ou ressentis.

De préférence, les données phénotypiques sont renseignées ou fournies par l'utilisateur du dispositif d'analyse selon l'invention (par exemple dans le cadre d'un questionnaire).

L'invention ne permet pas de, ni ne vise à, dresser un diagnostic thérapeutique.

L'utilisation ingénieuse de l'écart de température entre les deux seins et du profil de température sélectionné permet de s'affranchir de l'utilisation de données de référence comme c'est le cas dans l'état de la technique. Les données de référence utilisées dans l'état de la technique peuvent être des données standards ou physiologiques ou des données préalablement mesurées, pendant un laps de temps important (typiquement supérieur à 1 heure) sur la personne sur laquelle les données de température vont être mesurées pour déterminer la présence ou non d'une anomalie.

L'utilisation avantageuse de l'écart de température entre les deux seins repose sur le fait qu'il n'existe qu'une probabilité infime que des anomalies soient présentes sur chacun des seins.

La détermination de la présence ou non anomalie au moyen d'un modèle préalablement sélectionné confère également un avantage vis-à-vis des procédés de l'état de la technique. En effet, la sélection du modèle de traitement en fonction de l'écart de température calculé et du profil de température sélectionné permet d'utiliser un modèle de traitement correspondant au profil de la personne. En effet, les inventeurs ont observé l'existence d'un nombre limité de profils de température physiologique des seins. Ainsi, chaque sujet correspond à un profil de température donné. Ainsi, le traitement des données de température par un modèle de traitement particulier correspondant au profil du sujet/de la personne permet de limiter le nombre de faux positifs et la précision de l'information représentative déterminée.

A titre d'exemple, la sélection du modèle de traitement peut être réalisé par toute méthode statistique, par exemple de corrélation, connue de l'homme du métier.

Les effets techniques précités du procédé selon l'invention, en particulier mais pas uniquement l'utilisation d'un modèle traitement correspondant au profil du sujet, permettent de mettre en oeuvre la détermination d'une anomalie ou non à partir d'un temps d'acquisition de données de température limité. Typiquement, le procédé est mis en oeuvre à partir d'un temps d'acquisition de données de température compris entre 20 et 40 minutes, typiquement un temps d'acquisition de 30 minutes.

Selon un premier perfectionnement, le procédé comprend la détermination d'une signature statistique de l'écart de température entre les deux seins. De préférence, la signature statistique est déterminée à partir des données de température provenant de chacun des seins. La sélection du modèle de traitement de données approprié est opérée à partir de la signature statistique déterminée et du profil de température sélectionné.

La détermination de la signature statistique permet de diminuer le nombre de données de température et/ou le temps à partir duquel la sélection du modèle de traitement est réalisée. La détermination de la signature statistique permet de limiter les effets du bruit de mesure de la température.

Selon un deuxième perfectionnement, le procédé peut comprendre une étape de sous-échantillonnage du temps d'acquisition des données de température en sous-ensemble de données de température.

Le procédé comprend avantageusement, le calcul d'une température moyenne pour chaque sous-ensemble. Le calcul d'une température moyenne pour chaque sous ensemble permet de limiter les effets du bruit de mesure de la température et/ou de données de température manquantes ou aberrantes.

Selon un troisième perfectionnement, le procédé les données de température de chacun des seins comprennent n ensembles de données de température correspondant chacun à une zone distincte du sein, dit quadrant, où n est un entier. Selon le mode de réalisation, le procédé est mis en oeuvre à partir de 4 quadrants 21, 22, 23, 24 pour chaque sein. Les 4 quadrants sont notés QSI 21 (pour Quadrant Supérieur Intérieur), QSE 22 (pour quadrant Supérieur Extérieur), QIE 23 (pour Quadrant Inférieur Extérieur) et QII 24 (pour Quadrant Inférieur Intérieur).

Le mode de réalisation présenté comprenant une subdivision ou une segmentation de chacun des seins en quatre zones/quadrants est non-limitatives. Les données de température de chacun des seins pourraient être divisées en seulement deux ou trois quadrants. De manière avantageuse, les données de température de chacun des seins pourraient être divisées en cinq quadrants ou plus.

La compartimentation des données de température en quadrants 21, 22, 23, 24 permet, entre autres, de localiser l'anomalie dans un des quadrants 21, 22, 23, 24 lors de la détermination d'une anomalie. Toutefois, le calcul de l'écart de température ou de la détermination de la signature statistique est mis en oeuvre à partir des données de température provenant de chacun des n quadrants.

De préférence, l'écart de température entre les deux seins est calculé entre chaque quadrants 21, 22, 23, 24 correspondant. Autrement dit, un écart de température est calculé entre le quadrant 21 du sein gauche est le quadrant 21 du sein droit, un écart de température est calculé entre le quadrant 22 du sein droit est le quadrant 22 du sein gauche et cetera.

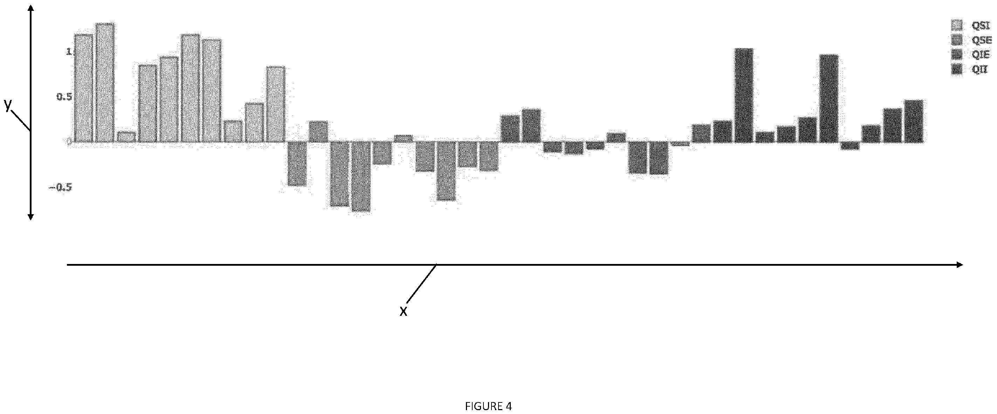

En référence à la FIGURE 4, et en référence aux premier, deuxième et troisième perfectionnement, il est illustré un exemple d'une signature statistique de l'écart de température entre les deux seins en fonction du temps. Sur l'axe des ordonnées (y) est illustrée de l'écart de température entre les deux seins. Sur l'axe des abscisses (x) est illustré les valeurs moyennées de l'écart de température entre les deux seins, pour chacun des 4 quadrants 21, 22, 23, 24, sur dix sous-ensembles.

On peut observer que l'écart de température entre les deux seins varie en fonction du temps. L'écart de température entre les deux seins varie au cours du temps. La signature statistique de l'écart de température entre les deux seins comprend une variation de l'écart de température entre les deux seins varie au cours du temps.

Selon un quatrième perfectionnement, l'écart de température et/ou la signature statistique est déterminée par comparaison des données de température d'un des seins, choisies comme données de température de référence, avec les données de température de l'autre sein. La comparaison peut être réalisée par combinaison linéaire, par exemple par soustraction.

Ainsi, le procédé permet de limiter la quantité de données nécessaire à la mise en oeuvre de l'invention, en particulier pour la sélection du modèle de traitement, tout en évitant d'avoir recours à des données standards ou physiologiques ou des données préalablement mesurées sur le sujet.

Selon un cinquième perfectionnement, le procédé comprend une étape consistant à déterminer :
- à partir des données de température, un ou des indicateurs représentatif(s) de la qualité des données mesurées, et/ou
- à partir de la signature statistique ou à partir de l'écart de température et du profil de température sélectionné, un ou des indicateurs représentatif(s) d'une tendance de l'écart de température entre les deux seins, et/ou
- à partir de la signature statistique ou à partir de l'écart de température, du ou des indicateurs représentatif(s) de la qualité des données mesurées et du profil de température sélectionné, un ou des indicateurs de fiabilité du modèle de traitement sélectionné, et/ou
- à partir du ou des indicateurs représentatif(s) de la qualité des données mesurées et/ou du ou des indicateurs de fiabilité du modèle de traitement sélectionné, un indicateur de fiabilité de l'information représentative de la présence ou non d'une anomalie, en particulier de fiabilité de l'information représentative de la présence ou non d'une anomalie dans un quadrant.

Chaque perfectionnement peut être mis en oeuvre individuellement.

Chaque perfectionnement peut être combiné avec l'un, plusieurs ou tous les autres perfectionnements.

Avantageusement, le traitement des données de température est mis en oeuvre par un modèle d'apprentissage statistique ou profond.

L'homme du métier saura choisir et adapter le modèle d'apprentissage statistique ou profond correspondant ou le plus adapté.

A titre d'exemple non limitatif, le modèle d'apprentissage statistique ou profond pourra être un réseau de neurones récurrent Long Short-Term Memory (LSTM). L'apprentissage débute par le dimensionnement des données pour rendre les données compatibles avec les couches LSTM. Le réseau de neurones récurrent est ensuite construit avec plusieurs couches LSTM. Une couche LSTM est composée d'une cellule LSTM qui est composée de plusieurs éléments internes : des portes d'entrée, de sortie, et d'oubli, ainsi que d'une cellule mémoire pour garder les caractéristiques importantes apprises lors des itérations et pour apprendre des séquences temporelles. Une couche dense avec activation sigmoïde est ajoutée à la fin pour la classification binaire pour la classification.

A titre d'exemple non limitatif, des techniques de régularisation telles que l'abandon (ou dropout en anglais) et la régularisation L2 (ou régression de crête
) sont appliquées pour prévenir le surapprentissage. Ce réseau neuronal permet de capturer les dépendances temporelles dans les séries de températures et d'effectuer une classification binaire pour détecter la présence des anomalies mammaires.

Selon l'invention, le procédé peut ne pas comprendre d'étape d'acquisition des données de températures.

Les données de températures peuvent être des données stockées.

Les données stockées peuvent provenir, par exemple, de mesures réalisées sur un ou un ensemble d'individus. Ainsi, le procédé peut être mis en oeuvre sur un ensemble de données stockées préalablement acquises indépendamment de la mise en oeuvre du procédé.

Les données stockées peuvent provenir, par exemple, de simulation(s). Ainsi, le procédé peut être mis en oeuvre sur un ensemble de données stockées indépendamment de la mise en oeuvre du procédé.

Avantageusement, le procédé comprend une étape d'apprentissage du modèle d'apprentissage statistique ou profond à partir d'une base de données d'apprentissage.

La base de données d'apprentissage comprend des données d'entrée consistant en un ensemble de données de température. Chaque ensemble de données de température comprend des données de température provenant de chacun des deux seins d'un même sujet.

La base de données d'apprentissage comprend, en outre, des données de sorties consistant en l'information représentative de la présence ou non d'une anomalie associée à chaque ensemble de données de température.

L'apprentissage du modèle d'apprentissage statistique ou profond permet d'enrichir les modèles de traitement de données de température.

Le procédé selon l'invention ne fournit pas un diagnostic thérapeutique.

Le procédé selon l'invention vise à fournir une information représentative de la présence ou non d'une anomalie dans les tissus mammaires.

Le procédé selon l'invention ne vise pas et ne permet pas de fournir un diagnostic thérapeutique. Le procédé selon l'invention fournie un indicateur informant l'individu de la pertinence ou non de consulter un praticien, et le cas échéant, de manière urgente (si l'indication de la présence d'une anomalie maligne est fournie) ou non (si l'indication de la présence d'une anomalie bénigne est fournie).

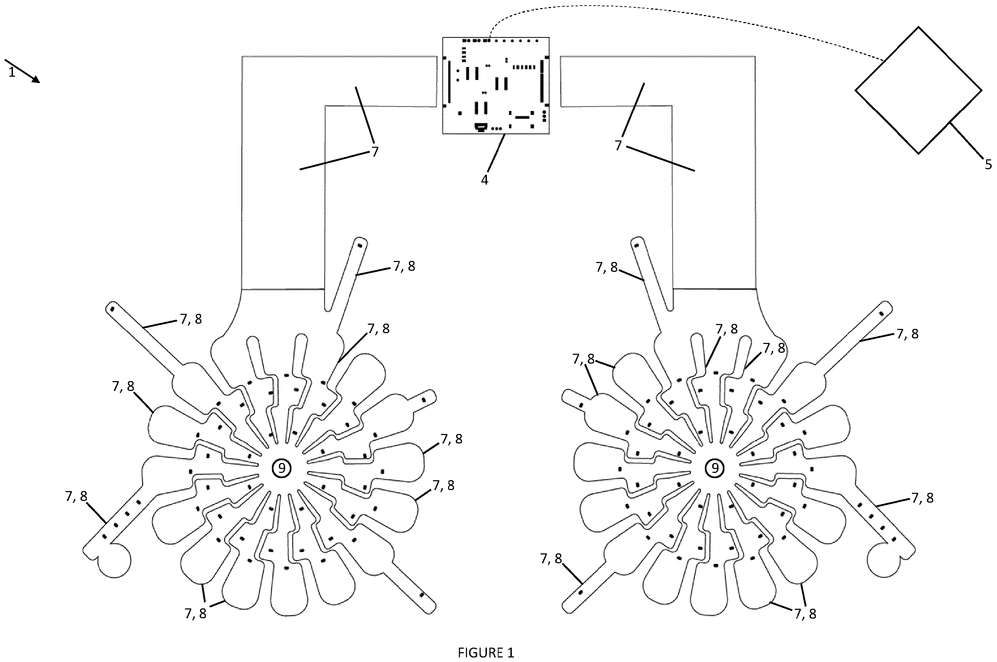

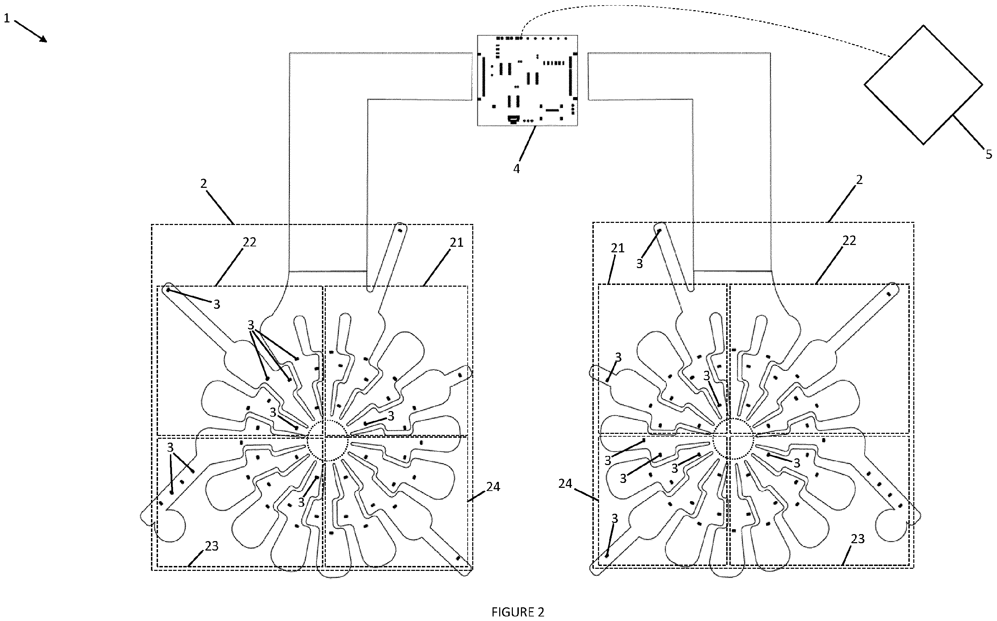

En référence aux FIGURES 1 et 2, il est également proposé un dispositif d'analyse par thermographie 1 pour la détection d'anomalies dans les tissus mammaires, dit dispositif 1.

Le dispositif 1 comprend deux réseaux 2 de capteurs de température 3 destinés à être en contact avec la peau d'un sein respectif. Chaque réseau 2 est agencés pour paver de manière homogène chacun des seins.

Selon le mode de réalisation non limitatif, les deux réseaux 2 de capteurs de température 3 sont symétriques l'un par rapport à l'autre. De préférence, les deux réseaux 2 de capteurs de température 3 sont symétriques par rapport à un plan médian ou sagittal de l'individu et/ou par rapport à un plan transversal de l'individu.

Le dispositif 1 comprend, en outre, une unité d'acquisition 4 de données de température au cours du temps, provenant des capteurs de température 3, agencée pour communiquer avec une unité de traitement 5. L'unité d'acquisition 4 et l'unité de traitement 5 sont connectées en filaire ou sans fil.

A titre d'exemple, selon le mode de réalisation non limitatif, le dispositif comprend 96 capteurs de température 3.

L'unité de traitement 5 est agencée et/ou configurée et/ou programmée pour mettre en oeuvre le procédé de détection d'anomalies dans les tissus mammaires par thermographie selon l'invention.

Le dispositif comprend un support flexible 7 d'un seul tenant.

Le support flexible 7 comprend les deux réseaux 2 de capteurs de température 3.

Le support flexible 7 comprend un ensemble de languettes 8 distinctes.

Les languettes 8 sont de géométrie actinomorphe.

Les languettes 8 comprennent le réseau 2 de capteurs de température 3.

Le support flexible 7 comprend une portion centrale 9 destinée à être positionnée sur la papille mammaire d'un sein.

Selon le mode de réalisation, au moins une partie des capteurs de température 5 sont répartis en losange et/ou en triangle et/ou en cercle sur le support flexible 7.

De préférence, les capteurs de température 5 sont répartis en cercles concentriques.

Selon le mode de réalisation, les capteurs de température 5 sont répartis en losange. Chacun des quatre sommets d'un losange correspond à un capteur de température 5. Pour chaque losange considéré et pour chaque capteur 5 de chaque languette 8, trois capteurs 5 sont disposés sur une languette 8 donnée et un capteur 5 est disposé sur une languette 8 adjacente à la languette 8 donnée.

Avantageusement, chaque languette 8 est séparée des deux languettes 8 adjacentes par deux fentes situées de part et d'autre de chaque languette 8.

De préférence, chaque languette 8 et/ou chaque fente, ou la bordure ou le bord ou le côté ou le pourtour séparant deux languettes 8 comprend des courbes et/ou une courbure et/ou n'est pas droit ou rectiligne ou plan.

De préférence, les capteurs de température 3, de préférence encore les capteurs de température 3 d'une même languette 8, ne sont pas alignés, en particulier radialement.

Ainsi, comparé à une répartition radiale et/ou axiale ou rectiligne des capteurs et/ou à une séparation et/ou à des fentes uniquement radiale et/ou axiale ou rectiligne entre deux languette 8, le pavage des capteurs de températures 3 et/ou l'agencement des languettes 8 permet de limiter le bruit de mesure de la température et/ou de données de température manquantes ou aberrantes et/ou les faux positifs.

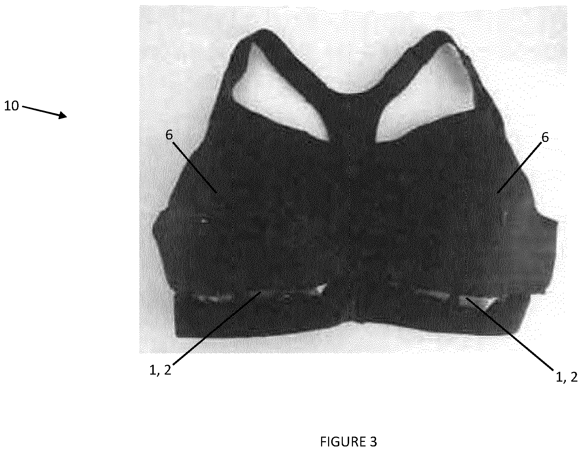

En référence à la FIGURE 3, il est également proposé un soutien-gorge 10 pour la détection d'anomalies dans les tissus mammaires par thermographie. Le soutien-gorge comprend le dispositif 1 selon l'invention.

Le soutien-gorge 10 comprend, en outre, deux bonnets 6. A l'intérieur de chaque bonnets 6 est disposé un des réseaux 2 de capteurs de température 3 respectif du dispositif 1.

Chacun des bonnets 6 est agencé pour isoler thermiquement les capteurs de température 3 de l'environnement situé à l'extérieur du soutien-gorge 10.

Bien sûr, l'invention n'est pas limitée aux exemples qui viennent d'être décrits et de nombreux aménagements peuvent être apportés à ces exemples sans sortir du cadre de l'invention.

En particulier toutes les variantes et tous les modes de réalisation décrits sont combinables entre eux si rien ne s'oppose à cette combinaison sur le plan technique.

## Revendications

1. Dispositif d'analyse par thermographie par contact cutané (1) pour la détection d'anomalies dans les tissus mammaires, dit dispositif (1) ; ledit dispositif comprenant :
- deux réseaux (2) de capteurs de température (3) étant destinés à être en contact avec la peau d'un sein respectif et étant agencés pour paver de manière homogène chacun des seins,
- une unité d'acquisition (4) de données de température au cours du temps, provenant des capteurs de température, agencée pour communiquer avec une unité de traitement (5), ladite unité de traitement étant agencée pour :
• calculer, à partir des données de température, un écart de température entre les deux seins en fonction du temps,
• sélectionner, à partir des données température, ou partir des données de température et de données phénotypiques, un profil de température correspondant aux données de température,
• sélectionner, à partir de l'écart de température en fonction du temps et du profil de température sélectionné, un modèle de traitement de données approprié,
• déterminer une information représentative de la présence ou non d'une anomalie par traitement des données de température avec le modèle de traitement sélectionné.

2. Dispositif (1) selon la revendication précédente, dans lequel l'unité de traitement est agencée pour, à partir des données de température :
- déterminer une signature statistique de l'écart de température entre les deux seins,
- sélectionner, à partir de la signature statistique de l'écart de température déterminée et du profil de température sélectionné, le modèle de traitement de données approprié.

3. Dispositif (1) selon la revendication 1 ou 2, dans lequel chaque réseau (2) de capteurs de température (3) comprend un support flexible (7) d'un seul tenant sur lequel sont disposés les capteurs de température, le support flexible comprend un ensemble de languettes (8) distinctes de géométrie actinomorphe et comprend une portion centrale (9) destinée à être positionnée sur la papille mammaire d'un sein.

4. Dispositif (1) selon la revendication précédente, dans lequel au moins une partie des capteurs de température sont répartis en losange sur le support flexible (7) ; chacun des quatre sommets d'un losange correspondant à un capteur de température, et, pour chaque losange considéré, trois capteurs de température sont disposés sur une languette donnée et un capteur de température est disposé sur une languette adjacente à la languette donnée.

5. Soutien-gorge (10) pour la détection d'anomalies dans les tissus mammaires par thermographie comprenant :
- le dispositif (1) selon l'une quelconque des revendications 3 à 4,
- deux bonnets (6) à l'intérieur desquels est disposé un réseau (2) de capteurs de température (3) respectif, chacun des deux réseaux de capteurs de température étant destiné à être en contact avec la peau d'un sein et étant agencé pour paver de manière homogène un sein ; chacun des bonnets est agencé pour isoler thermiquement les capteurs de température de l'environnement extérieur.

6. Procédé de détection d'anomalies dans les tissus mammaires par thermographie, dit procédé, ledit procédé comprend les étapes consistant à :
- calculer, au moyen d'une unité de traitement (5), un écart de température entre les deux seins en fonction du temps à partir de données de température de chacun des seins,
- sélectionner, au moyen de l'unité de traitement, à partir des données température, ou partir des données de température et de données phénotypiques, un profil de température correspondant aux données de tem pératu re,
- sélectionner, au moyen de l'unité de traitement, à partir de l'écart de température en fonction du temps et du profil de température sélectionné, un modèle de traitement de données de température approprié,
- déterminer une information représentative de la présence ou non d'une anomalie par traitement, au moyen de l'unité de traitement, des données de température avec le modèle de traitement sélectionné.

7. Procédé selon la revendication précédente, comprenant une étape d'acquisition, au moyen d'une unité d'acquisition (4), des données de température au cours du temps, mesurées au moyen de deux réseaux (2) de capteurs de température (3) en contact avec la peau d'un sein respectif et agencés pour former un pavage homogène de chacun des seins.

8. Procédé selon la revendication 6 ou 7, comprenant, en outre, une étape de détermination, à partir des données de température, d'une signature statistique de l'écart de température entre les deux seins ;
la sélection du modèle traitement de données de température est mise en oeuvre à partir de la signature statistique de l'écart de température déterminée et du profil de l'utilisateur.

9. Procédé selon l'une des revendications 6 à 8, dans lequel l'étape de sélection du modèle de traitement de données est mise en oeuvre à partir d'une variation de l'écart de température entre les deux seins dans le temps et du profil de température sélectionné.

10. Procédé selon l'une quelconque des revendications 8 ou 9, dans lequel l'écart de température et/ou la signature statistique est déterminée par comparaison des données de température d'un des seins, choisies comme données de température de référence, avec les données de température de l'autre sein.

11. Procédé selon l'une quelconque des revendications 8 à 10, dans lequel :
- les données de température de chacun des seins comprennent n ensembles de données de température correspondant chacun à une zone distincte du sein, dit quadrant, où n est un entier, et
- le calcul de l'écart de température et/ou la sélection du modèle de traitement de données et/ou la détermination de la signature statistique est mis en oeuvre à partir de l'ensemble des données de température provenant des n quadrants.

12. Procédé selon l'une quelconque des revendications 8 à 11, comprenant une étape consistant à déterminer, à partir de la signature statistique, un ou des indicateurs représentatif(s) d'une tendance de l'écart de température entre les deux seins ; l'étape de sélection du modèle de traitement de données de température est mise en oeuvre à partir du ou des indicateurs déterminés.

13. Procédé selon l'une quelconque des revendications 6 à 12, dans lequel le traitement des données de température est mis en oeuvre par un modèle d'apprentissage statistique ou profond.

14. Procédé selon la revendication précédente, comprenant une étape d'apprentissage du modèle sélectionné à partir d'une base de données d'apprentissage, ladite base de données d'apprentissage comprenant :
- des données d'entrée consistant en un ensemble de données de température, chaque ensemble comprenant des données de température provenant de chacun des deux seins d'un même sujet,
- des données de sorties consistant en l'information représentative de la présence ou non d'une anomalie associée à chaque ensemble de données de température.

15. Procédé selon la revendication précédente prise en combinaison avec l'une des revendications 8 à 12, dans lequel l'étape d'apprentissage est mise en oeuvre, indépendamment, pour chaque modèle de traitement, à partir des données d'apprentissage correspondant aux données de température.

16. Dispositif de traitement de données comprenant des moyens agencés et/ou programmés et/ou configurés pour mettre en oeuvre le procédé selon l'une quelconque des revendications 1 à 15.

17. Programme d'ordinateur comprenant des instructions qui, lorsque le programme est exécuté par un ordinateur, conduisent celui-ci à mettre en oeuvre le procédé selon l'une quelconque des revendications 1 à 15.

18. Support lisible par ordinateur comprenant des instructions qui, lorsqu'elles sont exécutées par un ordinateur, conduisent celui-ci à mettre en oeuvre le procédé selon l'une quelconque des revendications 1 à 15.
